# EUROPÄISCHE PATENTSCHRIFT

(11) **EP 1 197 566 B1**
(45) Veröffentlichungstag und Bekanntmachung des Hinweises auf die Patenterteilung: **18.05.2005**
(21) Anmeldenummer: 01123828.4
(22) Anmeldetag: 05.10.2001
(51) Int. Cl.: C12Q 1/68

(54) **Verfahren zur Sterilitätsprüfung von insbesondere flüssigen Medien**
Method for testing the sterility of liquid media
Procédé de vérification de la stérilité des milieux liquides

(30) Priorität: 11.10.2000 DE 10050135
(43) Veröffentlichungstag der Anmeldung: 17.04.2002
(73) Patentinhaber: BioChem Labor für biologische und chemische Analytik GmbH, 76185 Karlsruhe (DE)
(72) Erfinder:
(74) Vertreter: Lichti, Heiner, Dipl.-Ing.

(56) Entgegenhaltungen:
- US-A- 4 851 330
- US-A- 5 324 632
- US-A- 5 484 909
- HEINEMEYER T ET AL: "A sensitive method for the detection of murine C-type retroviruses" JOURNAL OF VIROLOGICAL METHODS, AMSTERDAM, NL, Bd. 63, 1997, Seiten 155-165, XP002092904 ISSN: 0166-0934
- WEZEL VAN A L ET AL: "DETECTION AND ELIMINATION OF CELLULAR NUCLEIC ACIDS IN BIOLOGICALS PRODUCED ON CONTINUOUS CELL LINES" DEVELOPMENTS IN BIOLOGICAL STANDARDIZATION, KARGER, BASEL, CH, Bd. 50, 1981, Seiten 59-69, XP001105736 ISSN: 0301-5149

## Beschreibung

Die Erfindung betrifft ein Verfahren zur Sterilitätsprüfung von insbesondere flüssigen Medien, wie pharmazeutischen und/oder medizinischen Präparaten.

Zur Untersuchung der Sterilität von Medien, die höchsten Anforderungen an die Hygiene genügen müssen, z. B. flüssigen, emulgierten oder gelösten pharmazeutischen und medizinischen Präparaten, Wirkstoffen, Injektions- und Infusionslösungen, Augentropfen oder dergleichen, aber auch chirurgischen Implantaten, ist es bekannt, das zu untersuchende Medium unter aseptischen Bedingungen unter Einwirken von Nährmedien über mehrere Tage zu inkubieren. Im Falle einer nach der Inkubation auftretenden visuell wahrnehmbaren Eintrübung durch Verkeimung werden Proben entnommen und zum Zwecke der Identifizierung weiter angereichert. Anschließend werden die angereicherten Keime mittels mikrobiologischer Verfahren bestimmt. Nach Bestätigung einer Verkeimung wird die untersuchte Probe als nicht steril deklariert.

Nachteilig ist einerseits der hohe Zeitbedarf sowohl für die Inkubation als auch - bei Auftreten einer Verkeimung - für die weitergehende Anreicherung und mikrobiologische Bestimmung der Keime, was zu hohen Lagerkosten und großem Platzbedarf für die untersuchten Proben und im Falle des Nachweises von Keimen zu einer erhöhten Ausschußproduktion führt. Andererseits werden mit dem bekannten Verfahren nur diejenigen Vertreter von Bakterien, Hefen, Pilzen etc. erfaßt, denen mit den jeweils eingesetzten Nährmedien geeignete Wachstumsbedingungen zur Verfügung gestellt werden, so daß ein erheblicher Anteil von Mikroorganismen, wie bestimmte Bakterienarten, insbesondere Mycoplasmen, auf diese Weise nicht nachgewiesen werden können.

Ferner ist die Polymerase-Kettenreaktion (polymerase chain reaction, PCR) bekannt, welche zur selektiven DNA-Synthese von zwischen zwei kurzen einsträngigen synthetischen Oligonucleotiden (Primern) angeordneten DNA-Sequenzen unter Einsatz von DNA-Polymerase und der Desoxynucleosintriphosphate (dNTP) Desoxyadenintriphosphat (dATP), Desoxythymintriphosphat (dTTP), Desoxycytosintriphosphat (dCTP) und Desoxyguanintriphosphat (dGTP) verwendet wird. Dieses gentechnische Verfahren ermöglicht eine selektive Amplifikation bestimmter DNA-Abschnitte, indem die zu amplifizierende DNA (Doppelstrang-DNA) thermisch zu Einzelstrang-DNA denaturiert wird, so daß die zum Anfang und Ende des zu amplifizierenden DNA-Bereichs komplementären Primer an die Einzelstrang-DNA binden (Annealing) und mit diesem Bereich hybridisiert werden. Die hybridisierten Primer dienen so als Startpunkte für die z. B. aus *Thermus aquaticus* gewonnene hitzestabile DNA-Polymerase zur spezifischen Synthese neuer Doppelstränge aus den Desoxynucleosintriphosphaten. In der Regel werden jeweils am Sinnstrang und am Gegenstrang bindende Primer eingesetzt, so daß der zwischen den beiden Primern angeordnete DNA-Abschnitt verdoppelt wird. Dieser Prozeß kann beliebig oft wiederholt werden, wobei mit jedem Zyklus jeweils eine Verdopplung der zu amplifizierenden DNA erreicht wird. Auf diese Weise ist eine exponentielle Amplifikation möglich, da bei jedem Zyklus auch die bei den vorherigen Zyklen gebildeten DNA-Stränge als Matrize zur Verfügung stehen. PCR-basierten Verfahren zum Nachweis von Mikroorganismen sind in US 3244632 und Heinemeyer et al. (1997; J. Virol., 63, 155-165) beschrieben.

Der Erfindung liegt die Aufgabe zugrunde, ein schnelles und preiswertes Verfahren zur Sterilitätsprüfung von insbesondere flüssigen Medien, wie pharmazeutischen und/oder medizinischen Präparaten vorzuschlagen, welches unter Vermeidung falsch-positiver Ergebnisse zum Nachweis im wesentlichen sämtlicher Mikroorganismen sowie Viren geeignet ist.

Erfindungsgemäß wird diese Aufgabe mit einem Verfahren der eingangs genannten Art gelöst, welches durch folgende Schritte gekennzeichnet ist:
a) Aufkonzentrieren von gegebenenfalls in dem Medium enthaltenen Mikroorganismen unter Bildung eines Konzentrates;
b) Spalten der im Konzentrat gegebenenfalls enthaltenen freien Nucleinsäuren durch Zusetzen wenigstens einer Desoxyribonuclease (DNAse);
c) Extrahieren der Desoxyribonucleinsäure (DNA) und der Ribonucleinsäure (RNA) der im Konzentrat gegebenenfalls enthaltenen Mikroorganismen unter gleichzeitigem Zerstören sowohl der Zellwände der Mikroorganismen als auch der zugesetzten DNAse;
d) chromatographisches Isolieren der DNA;
e) Anreichern der DNA durch Polymerase-Kettenreaktion (polymerase chain reaction, PCR) unter Einsatz von Primern, die sich aus hochkonservierten Regionen ableiten, und/oder zufälligen Primern (Random-Primern) und/oder willkürlichen Primern (Arbitrary-Primern);
f) qualitatives Bestimmen der mittels PCR angereicherten DNA.

Die Vorteile des erfindungsgemäßen Verfahrens liegen einerseits in einer gegenüber dem Stand der Technik erheblich verkürzten Untersuchungsdauer von etwa ein bis zwei Tagen, da sowohl die Inkubation zur Kultivierung der in dem zu untersuchenden Medium gegebenenfalls enthaltenen Mikroorganismen als auch die weitergehende Anreicherung im Falle eines positiven Befundes entbehrlich werden. Ferner werden durch den Einsatz geeigneter, insbesondere verschiedener Primer bei der Polymerase-Kettenreaktion nahezu sämtliche Mikroorganismen erfaßt, wobei aufgrund der hohen Amplifikationrate der PCR auch die DNA einzelner Zellen erfaßt wird, so daß eine größtmögliche Empfindlichkeit des Verfahrens sichergestellt ist. Durch die Spaltung von gegebenenfalls vorhandenen freien Nucleinsäuren, die sich nicht in vermehrungsfähigen Mikroorganismen befinden, werden durch Zusetzen wenigstens einer DNAse falsch-positive Ergebnisse zuverlässig vermieden. Schließlich können mit dem erfindungsgemäßen Verfahren auch Viren erfaßt werden.

Weitere Merkmale und Vorteile des erfindungsgemäßen Verfahrens ergeben sich aus der nachfolgenden Beschreibung einer bevorzugten Ausführungsform.

Zur Untersuchung der Sterilität einer pharmazeutischen Lösung wird diese z. B. durch Zentrifugieren, Sterilfiltrieren oder dergleichen aufkonzentriert. Eventuell in der Lösung enthaltene Mikroorganismen werden auf diese Weise angereichert und in einem Desoxyribonuclease-Puffer (DNAse-Puffer) aufgenommen und eine DNAse, insbesondere eine Endonuclease, wie Desoxyribonuclease I (DNAse I), zugesetzt. Die DNAse-Behandlung dient ausschließlich der Spaltung von gegebenenfalls im Konzentrat enthaltenen freien Nucleinsäuren aus nicht vermehrungsfähigen Mikroorganismen in Mono- bzw. Dinucleotide. Diese freien, extrazellularen Nucleinsäuren bzw. DNA-Fragmente, die z. B. aus abgetöteten Zellen stammen, würden andernfalls bei der PCR falsch-positive Ergebnisse hervorrufen, da bei der PCR unabhängig von lebenden oder toten Mikroorganismen sämtliche in der Lösung befindlichen Desoxyribonucleinsäuren amplifiziert werden. Somit würde die untersuchte Probe auch nur bei Vorhandensein nicht vermehrungsfähiger Mikroorganismen als nicht steril befunden werden.

Anschließend wird die DNA und die RNA der im Konzentrat gegebenenfalls enthaltenen Mikroorganismen oder Viren unter gleichzeitigem Zerstören sowohl der Zellwände der Mikroorganismen als auch der zugesetzten DNAse extrahiert. Hierfür ist beispielsweise vorgesehen, daß zum Extrahieren der DNA und der RNA von im Konzentrat gegebenenfalls enthaltenen Bakterien Lysozyme (Peptidoglykan-N-Acetylmuramoylhydrolasen) und/oder Lysostaphin (Peptidoglykan-Endopeptidase) bzw. zum Extrahieren der DNA und der RNA von im Konzentrat gegebenenfalls enthaltenen Pilzen, Hefen und anderen Eukaryonten Lyticasen und/oder Proteinasen, insbesondere Proteinase-K, zugesetzt werden, wobei insbesondere die Proteinase-K die zuvor zur Spaltung freier Nucleinsäuren zugesetzte DNAse enzymatisch beseitigt. Um die gesamte DNA und RNA der im Konzentrat gegebenenfalls vorkommenden Prokaryonten und Eukaryonten zu extrahieren, ist alternativ oder zusätzlich vorgesehen, daß chaotrope Salze, insbesondere Guanidinhydrochlorid und/oder Guanidinisothiocyanat, zugesetzt werden und/oder eine Hitzebehandlung durchgeführt wird und/oder feine Partikel aus einem inerten Material, insbesondere Glasbeads, zugesetzt und diese mechanisch agitiert werden.

Die derart extrahierte DNA und vorzugsweise auch die RNA werden sodann chromatographisch isoliert, was auf an sich bekannte Weise mittels Adsorptionschromatographie, insbesondere Silicagel-Adsorptionschromatographie erfolgen kann.

Während grundsätzlich auch nur die extrahierte DNA isoliert und als Ausgangsmaterial für die PCR eingesetzt werden kann, ist in bevorzugter Ausführung vorgesehen, daß sowohl die DNA als auch die RNA z. B. mittels Silicagel-Adsorptionschromotographie isoliert werden und die RNA durch Zusetzen wenigstens einer reversen Transkriptase (Revertase) in komplementäre DNA (cDNA) transkribiert wird. Auf diese Weise wird die Empfindlichkeit des erfindungsgemäßen Verfahrens erheblich erhöht, indem neben der DNA auch die naturgemäß in weitaus größeren Mengen in Prokaryonten und Eukaryonten enthaltene RNA der PCR in Form von cDNA zugänglich gemacht und so ebenfalls amplifiziert wird.

Anschließend wird die isolierte DNA und vorzugsweise auch die isolierte RNA, nachdem sie in cDNA transkribiert worden ist, mittels PCR amplifiziert, wobei insbesondere mehrere verschiedene Primer zum Einsatz kommen, um im wesentlichen die gesamte isolierte DNA unabhängig von ihrer Herkunft zu amplifizieren. Hierfür ist einerseits vorgesehen, daß zum Nachweis von Eukaryonten, wie Hefen, Pilzen und Eubakterien, solche DNA-Oligonucleotide eingesetzt werden, welche zu hochkonservierten rRNA-Sequenzen (ribosomale RNA-Sequenzen) komplementär sind, insbesondere Primer, die sich von den hochkonservierten 16S-rRNA- und/oder 18S-rRNA-Sequenzen ableiten. Des weiteren kommen solche Primer in Frage, die sich von hochkonservierten snRNA-Sequenzen (kleine Kern-RNA-Sequenzen), und/oder Primer, die sich von dem hochkonservierten Poly-A-Schwanz von mRNA (endständige Poly-Adenin-Gruppe der Messenger-RNA) ableiten, vorzugsweise Oligo(dT)₁₄-Primer.

Zum Nachweis von Prokaryonten, wie Bakterien, andererseits ist neben dem Einsatz von sich aus hochkonservierten Regionen ableitenden Primern (in diesem Fall insbesondere zu 16S-rRNA komplementäre Primer) der Einsatz von zufälligen Primern (Random-Primern) und/oder willkürlichen Primern (Arbitrary-Primern) vorgesehen, wobei im Falle von Arbitrary-Primern mit Vorzug eine Reihe willkürlich gewählter Oligonucleotide mit bis zu zehn Basenpaaren eingesetzt werden, die an nicht bekannte Regionen des DNA-Genoms binden können. Im Falle von Random-Primern werden bevorzugt hochdegenerierte, im wesentlichen vollständig permutierte Oligonucleotide eingesetzt, die sowohl an bekannte als auch an noch nicht entschlüsselte DNA-Sequenzen von Prokaryonten, wie Archebakterien, Mycoplasmen etc., aber auch Eukaryonten und Viren binden können. Durch die Kombination einer Mehrzahl geeigneter Primer wird die Amplifikation von DNA nahezu beliebiger Herkunft sichergestellt und somit die Detektion beliebiger mikrobiologischer Spurenkontaminationen der zu untersuchenden Lösung einschließlich Viren ermöglicht.

Die Polymerase-Kettenreaktion (PCR) wird zweckmäßig in 20 bis 50, insbesondere 30 bis 40 aufeinanderfolgenden Zyklen durchgeführt, wobei jeder Zyklus die Denaturierung der zu amplifizierenden DNA, die Bindung der Primer an die denaturierte Einzelstrang-DNA (Annealing) und die Synthese von Komplementärsträngen aus den Desoxynucleosintriphosphaten mittels DNA-Polymerase umfaßt.

Die qualitative Bestimmung der mittels PCR angereicherten DNA erfolgt z. B. auf bekannte Weise durch Auftrennen derselben mittels Gelelektrophorese, insbesondere Agarose-Gelelektrophorese, und Einlagerung wenigstens eines Fluoreszenz-Farbstoffs, der durch UV-Bestrahlung nachgewiesen werden kann. Als Fluoreszenz-Farbstoff kommt insbesondere 3,8-Diamino-5-ethyl-6-phenylphenanthridiniumbromid (Homidiumbromid, Ethidiumbromid) in Frage.

Das erfindungsgemäße Verfahren gewährleistet einen schnellen, kostengünstigen und sensiblen Nachweis nahezu beliebiger Mikroorganismen und Viren aus flüssigen oder viskosen Medien und Lösungen, die höchsten Anforderungen an die Hygiene genügen müssen, wie beispielsweise pharmazeutischen und medizinischen Präparaten. Es ist selbstverständlich auch zur Sterilitätsprüfung von festen und gasförmigen Medien geeignet, indem diese z. B. extrahiert werden und das Extrakt auf vorstehend beschriebene Weise untersucht wird.

## Patentansprüche

1. Verfahren zur Sterilitätsprüfung von insbesondere flüssigen Medien, wie pharmazeutischen und/oder medizinischen Präparaten, **gekennzeichnet durch** folgende Schritte:
a) Aufkonzentrieren von gegebenenfalls in dem Medium enthaltenen Mikroorganismen unter Bildung eines Konzentrates;
b) Spalten der im Konzentrat gegebenenfalls enthaltenen freien Nucleinsäuren **durch** Zusetzen wenigstens einer Desoxyribonuclease (DNAse);
c) Extrahieren der Desoxyribonucleinsäure (DNA) und der Ribonucleinsäure (RNA) der im Konzentrat gegebenenfalls enthaltenen Mikroorganismen unter gleichzeitigem Zerstören sowohl der Zellwände der Mikroorganismen als auch der zugesetzten DNAse;
d) chromatographisches Isolieren der DNA;
e) Anreichern der DNA **durch** Polymerase-Kettenreaktion (polymerase chain reaction, PCR) unter Einsatz von Primern, die sich aus hochkonservierten Regionen ableiten, und/oder zufälligen Primern (Random-Primern) und/oder willkürlichen Primern (Arbitrary-Primern);
f) qualitatives Bestimmen der mittels PCR angereicherten DNA.

2. Verfahren nach Anspruch 1, **dadurch gekennzeichnet, daß** die Mikroorganismen durch Zentrifugieren mechanisch aufkonzentriert werden.

3. Verfahren nach Anspruch 1, **dadurch gekennzeichnet, daß** die Mikroorganismen durch Sterilfiltrieren aufkonzentriert werden.

4. Verfahren nach einem der Ansprüche 1 bis 3, **dadurch gekennzeichnet, daß** die im Konzentrat enthaltenen freien Nucleinsäuren durch Zusetzen wenigstens einer Endonuclease, insbesondere Desoxyribonuclease I (DNAse I), in Mono- bzw. Dinucleotide gespalten werden.

5. Verfahren nach einem der Ansprüche 1 bis 4, **dadurch gekennzeichnet, daß** zum Extrahieren der DNA und der RNA von im Konzentrat enthaltenen Bakterien Lysozyme (Peptidoglykan-N-Acetylmuramoylhydrolasen) und/oder Lysostaphin (Peptidoglykan-Endopeptidase) zugesetzt werden.

6. Verfahren nach einem der Ansprüche 1 bis 5, **dadurch gekennzeichnet, daß** zum Extrahieren der DNA und der RNA von im Konzentrat enthaltenen Pilzen, Hefen oder anderen Eukaryonten Lyticasen und/oder Proteinasen, insbesondere Proteinase-K, zugesetzt werden.

7. Verfahren nach einem der Ansprüche 1 bis 6, **dadurch gekennzeichnet, daß** zum Extrahieren der DNA und der RNA von im Konzentrat enthaltenen Prokaryonten und Eukaryonten chaotrope Salze, insbesondere Guanidinhydrochlorid und/oder Guanidinisothiocyanat, zugesetzt werden.

8. Verfahren nach einem der Ansprüche 1 bis 7, **dadurch gekennzeichnet, daß** zum Extrahieren der DNA und der RNA von im Konzentrat enthaltenen Prokaryonten und Eukaryonten eine Hitzebehandlung durchgeführt wird.

9. Verfahren nach einem der Ansprüche 1 bis 8, **dadurch gekennzeichnet, daß** zum Extrahieren der DNA und der RNA von im Konzentrat enthaltenen Prokaryonten und Eukaryonten feine Partikel aus einem inerten Material, insbesondere Glasbeads, zugesetzt und diese mechanisch agitiert werden.

10. Verfahren nach einem der Ansprüche 1 bis 9, **dadurch gekennzeichnet, daß** zusätzlich zu der DNA auch die RNA chromatographisch isoliert und durch Zusetzen wenigstens einer reversen Transkriptase (Revertase) in cDNA transkribiert wird;

11. Verfahren nach einem der Ansprüche 1 bis 10, **dadurch gekennzeichnet, daß** die DNA und gegebenenfalls die RNA mittels Adsorptionschromatographie, insbesondere Silicagel-Adsorptionschromatographie, isoliert werden.

12. Verfahren nach einem der Ansprüche 1 bis 11, **dadurch gekennzeichnet, daß** bei der Polymerase-Kettenreaktion Primer eingesetzt werden, die sich von der hochkonservierten 16S-rRNA-Sequenz ableiten.

13. Verfahren nach einem der Ansprüche 1 bis 12, **dadurch gekennzeichnet, daß** bei der Polymerase-Kettenreaktion Primer eingesetzt werden, die sich von der hochkonservierten 18S-rRNA-Sequenz ableiten.

14. Verfahren nach einem der Ansprüche 1 bis 13, **dadurch gekennzeichnet, daß** bei der Polymerase-Kettenreaktion Primer eingesetzt werden, die sich von hochkonservierter snRNA-Sequenzen ableiten.

15. Verfahren nach einem der Ansprüche 1 bis 14, **dadurch gekennzeichnet, daß** bei der Polymerase-Kettenreaktion Primer eingesetzt werden, die sich von dem hochkonservierten Poly-A-Schwanz von mRNA ableiten, insbesondere Oligo (dT)₁₄-Primer.

16. Verfahren nach einem der Ansprüche 1 bis 15, **dadurch gekennzeichnet, daß** bei der Polymerase-Kettenreaktion Arbitrary-Primer mit bis zu zehn Basenpaaren eingesetzt werden.

17. Verfahren nach einem der Ansprüche 1 bis 16, **dadurch gekennzeichnet, daß** bei der Polymerase-Kettenreaktion hochdegenerierte, im wesentlichen vollständig permutierte Random-Primer eingesetzt werden.

18. Verfahren nach einem der Ansprüche 1 bis 17, **dadurch gekennzeichnet, daß** die Polymerase-Kettenreaktion in 20 bis 50, insbesondere 30 bis 40 aufeinanderfolgenden Zyklen durchgeführt wird.

19. Verfahren nach einem der Ansprüche 1 bis 18, **dadurch gekennzeichnet, daß** zur qualitativen Bestimmung der angereicherten DNA diese mittels Gelelektrophorese, insbesondere Agarose-Gelelektrophorese, aufgetrennt und nach Einlagerung wenigstens eines Fluoreszenz-Farbstoffes mittels UV-Bestrahlung nachgewiesen wird.

20. Verfahren nach Anspruch 19, **dadurch gekennzeichnet, daß** als Fluoreszenz-Farbstoff 3,8-Diamino-5-ethyl-6-phenylphenanthridiniumbromid eingesetzt wird.

## Claims

1. A method for the examination of the sterility of particularly liquid media, e.g. pharmaceuticals and/or medical preparations, the method comprising the steps of:
a) concentrating microorganisms which may be contained in the medium, thereby forming a concentrate;
b) dissociating free nucleic acids which may be contained in said concentrate by adding at least one desoxyribonuclease (DNAse);
c) extracting desoxyribonucleic acid (DNA) and ribonuleic acid (RNA) from the microorganisms which may be contained in said concentrate thereby simultaneously destroying cell walls of the microorganisms and said added DNAse;
d) chromatographically isolating said DNA;
e) concentrating said DNA through polymerase chain reaction (PCR) thereby using primers which are derived from highly-conserved regions and/or random primers and/or arbitrary primers; and
f) qualitatively determining said DNA concentrated through PCR.

2. The method of claim 1, **characterised in that** the microorganisms are concentrated using mechanical centrifugation.

3. The method of claim 1, **characterised in that** the microorganisms are concentrated using sterile filtration.

4. The method of any one of claims 1 to 3, **characterised in that** said free nucleic acids contained in said concentrate are dissociated into at least one of mono and dinucleotides by adding at least one endonuclease, particularly desoxyribonuclease I (DNAse I).

5. The method of any one of claim 1 to 4, **characterised in that** at least one of lysozymes (peptidoglycan-N-acetyl-muramoylhydrolases) and lysostaphin (peptidoglykan-endopeptidase) is added for extracting DNA and RNA from bacteria contained in said concentrate.

6. The method of any one of claims 1 to 5, **characterised in that** at least one of lyticases and proteinases, particularly proteinase-K, is added for extracting DNA and RNA from at least one of fungi, yeast and other eukaryotes contained in said concentrate.

7. The method of any one of claims 1 to 6, **characterised in that** at least one of chaotropic salts, particularly guanidine hydrochloride and/or guanidine isothiocyanate, are added for extracting DNA and RNA from prokaryotes and eukaryotes contained in said concentrate.

8. The method of any one of claims 1 to 7, **characterised in that** heat treatment is carried out for extracting DNA and RNA from prokaryotes and eukaryotes contained in said concentrate.

9. The method of any one of claims 1 to 8, **characterised in that** fine particles of an inert material, particularly glass beads, are added and mechanically agitated for extracting DNA and RNA from prokaryotes and eukaryotes contained in said concentrate.

10. The method of any one of claims 1 to 9, further comprising chromatographically isolating not only DNA, but also RNA and transcribing said RNA into cDNA by adding at least one reverse transcriptase (revertase).

11. The method of any one of claims 1 to 10, **characterised in that** at least one of DNA and RNA is isolated using adsorption chromatography, particularly silicagel adsorption chromatography.

12. The method of any one of claims 1 to 11, **characterised in that** said primers used in said polymerase chain reaction are derived from a highly-conserved 16S-rRNA sequence.

13. The method of any one of claims 1 to 12, **characterised in that** said primers used in said polymerase chain reaction are derived from a highly-conserved 18S-rRNA sequence.

14. The method of any one of claims 1 to 13, **characterised in that** said primers used in said polymerase chain reaction are derived from a highly-conserved snRNA sequence.

15. The method of any one of claims 1 to 14, **characterised in that** said primers used in said polymerase chain reaction are derived from a highly-conserved poly-A-tail of mRNA, particularly Oligo(dT)₁₄ primers.

16. The method of any one of claims 1 to 15, **characterised in that** arbitrary primers of up to ten base pairs are used for said polymerase chain reaction.

17. The method of any one of claims 1 to 16, **characterised in that** highly-degenerated, substantially completely permuted random primers are used for said polymerase chain reaction.

18. The method of any one of claims 1 to 17, **characterised in that** said polymerase chain reaction is carried out in 20 to 50 successive cycles, particularly in 30 to 40 successive cycles.

19. The method of any one of claims 1 to 18, **characterised in that** for said qualitative determination of said concentrated of DNA, said DNA is dissociated using gel electrophoresis, particularly agarose gel electrophoresis, and detected using UV radiation after deposition of at least one fluorescent pigment.

20. The method of claim 19, **characterised in that** 3,8-diamino-5-ethyl-6-phenyl-phenanthridiniumbromide is used as said fluorescent pigment.

## Revendications

1. Procédé de vérification du caractère stérile en particulier de milieux liquides tels que des préparations pharmaceutiques et/ou médicinales, **caractérisé par** les étapes suivantes :
a) concentration des micro-organismes contenus dans le milieu le cas échéant par formation d'un concentré ;
b) décomposition des acides nucléiques libres contenus dans le concentré le cas échéant par addition d'au moins une désoxyribonucléase (DNase) ;
c) extraction de l'acide désoxyribonucléique (ADN) et de l'acide ribonucléique (ARN) des micro-organismes contenus, le cas échéant, dans le concentré, avec destruction simultanée tant des membranes cellulaires des micro-organismes que des DNases ajoutées ;
d) isolation chromatographique des ADN ;
e) amplification de l'ADN par réaction en chaîne de la polymérase, RCP (polymerase chain reaction, PCR) en mettant en oeuvre des amorces dérivant de régions hautement conservées, et/ou des amorces aléatoires (Random-Primers), et/ou des amorces arbitraires (Arbitrary-Primers) ;
f) détermination qualitative de l'ADN amplifié par RCP.

2. Procédé selon la revendication 1, **caractérisé en ce que** les micro-organismes sont concentrés mécaniquement par centrifugation.

3. Procédé selon la revendication 1, **caractérisé en ce que** les micro-organismes sont concentrés par filtration stérile.

4. Procédé selon l'une quelconque des revendications 1 à 3, **caractérisé en ce que** les acides nucléiques contenus dans le concentré sont décomposés en mononucléotides ou dinucléotides par addition d'au moins une endonucléase, en particulier la désoxyribonucléase I (DNase I).

5. Procédé selon l'une quelconque des revendications 1 à 4, **caractérisé en ce que** pour extraire l'ADN et l'ARN des bactéries contenues dans le concentré, on ajoute des lysozymes (peptidoclycan-N-acetylmuramoylhydrolase) et/ou de la lysostaphine (peptidoglycan-endopeptidase).

6. Procédé selon l'une quelconque des revendications 1 à 5, **caractérisé en ce que** pour extraire l'ADN et l'ARN des champignons, levures ou autres eucaryotes contenus dans le concentré, on ajoute des lyticases et/ou des protéinases, en particulier de la protéinase K.

7. Procédé selon l'une quelconque des revendications 1 à 6, **caractérisé en ce que** pour extraire l'ADN et l'ARN des procaryotes et des eucaryotes contenus dans le concentré, on ajoute des sels chaotropes, en particulier de l'hydrochlorure de guanidine et/ou de l'isothiocyanate de guanidine.

8. Procédé selon l'une quelconque des revendications 1 à 7, **caractérisé en ce que** pour extraire l'ADN et l'ARN de procaryotes et d'eucaryotes contenus dans le concentré on procède à un traitement par la chaleur.

9. Procédé selon l'une quelconque des revendications 1 à 8, **caractérisé en ce que** pour extraire l'ADN et l'ARN de procaryotes et d'eucaryotes contenus dans le concentré, on ajoute de fines particules d'un matériau inerte, en particulier des billes de verre et on les agite mécaniquement.

10. Procédé selon l'une quelconque des revendications 1 à 9, **caractérisé en ce qu'**en plus de l'ADN on isole également l'ARN par chromatographie et on le transcrit en ADNc par addition d'au moins une transcriptase inverse (invertase).

11. Procédé selon l'une quelconque des revendications 1 à 10, **caractérisé en ce que** l'on isole l'ADN et le cas échéant l'ARN par chromatographie d'adsorption, en particulier par chromatographie d'adsorption au gel de silice.

12. Procédé selon l'une quelconque des revendications 1 à 11, **caractérisé en ce que** dans la réaction en chaîne de la polymérase on met en oeuvre des amorces hautement conservées dérivant de la séquence ARNr 16S.

13. Procédé selon l'une quelconque des revendications 1 à 12, **caractérisé en ce que** dans la réaction en chaîne de la polymérase on met en oeuvre des amorces hautement conservées dérivant de la séquence ARNr 18S.

14. Procédé selon l'une quelconque des revendications 1 à 13, **caractérisé en ce que** dans la réaction en chaîne de la polymérase on met en oeuvre des amorces hautement conservées dérivant de séquences ARNsn.

15. Procédé selon l'une quelconque des revendications 1 à 14, **caractérisé en ce que** dans la réaction en chaîne de la polymérase on met en oeuvre des amorces dérivant de la queue de poly A d'ARNm hautement conservée, en particulier des amorces oligo(dT)₁₄.

16. Procédé selon l'une quelconque des revendications 1 à 15, **caractérisé en ce que** dans la réaction en chaîne de la polymérase on met en oeuvre des amorces arbitraires comprenant jusqu'à dix paires basiques.

17. Procédé selon l'une quelconque des revendications 1 à 16, **caractérisé en ce que** dans la réaction en chaîne de la polymérase on met en oeuvre des amorces aléatoires hautement dégénérées, pour l'essentiel entièrement permutées.

18. Procédé selon l'une quelconque des revendications 1 à 17, **caractérisé en ce que** la réaction en chaîne de la polymérase est effectuée en 20 à 50, plus particulièrement en 30 à 40 cycles consécutifs.

19. Procédé selon l'une quelconque des revendications 1 à 18, **caractérisé en ce que** pour déterminer qualitativement l'ADN amplifié, on le sépare par électrophorèse sur gel, en particulier par électrophorèse sur gel d'agarose et on l'identifie par rayonnement UV après stockage d'au moins une couleur fluorescente.

20. Procédé selon la revendication 19, **caractérisé en ce que** l'on utilise comme couleur fluorescente du bromure de 3,8-diamino-5-éthyl-6-phényl-phénanthridinium.
